# EUROPEAN PATENT SPECIFICATION

(11) **EP 1 450 702 B1**
(45) Date of publication and mention of the grant of the patent: **13.06.2012**
(21) Application number: 02802879.3
(22) Date of filing: 07.11.2002
(51) Int. Cl.: A61B 17/32

(54) **AN ULTRASONIC CLAMP COAGULATOR APPARATUS HAVING AN IMPROVED CLAMPING END-EFFECTOR**
ULTRASCHALL-KLEMMKOAGULATORGERÄT MIT VERBESSERTEM KLEMM-ENDEFFEKTOR
APPAREIL COAGULATEUR ULTRASONORE A CLAMP AVEC AMELIORATION PORTANT SUR UN EFFECTEUR DE CLAMPAGE D'EXTREMITE

(30) Priority: 07.11.2001 US 338271 P
(43) Date of publication of application: 01.09.2004
(73) Proprietor: ETHICON ENDO-SURGERY, INC., Cincinnati, Ohio 45242 (US)
(72) Inventor: CRAIG, Wayne, H., Cincinnati Ohio 45249 (US); CUMMINGS, John, Maderia, OH 45236 (US); GIORDANO, James, R., Milford, OH 45150 (US); HOUSER, Kevin, Springboro, OH 45066 (US); NEUENFELDT, Steve, K., Cincinnati, OH 45249 (US); SCHWEMBERGER, Richard, Cincinnati, OH 45247 (US); YATES, David, West Chester, OH 45069 (US); WAMPLER, Scott, West Chester, OH 45069 (US)
(74) Representative: Tunstall, Christopher Stephen
(86) International application number: PCT/US2002/035843
(87) International publication number: WO 2003/039429

(56) References cited:
- EP-A1- 0 830 845
- WO-A-99/35982
- WO-A2-00/64358
- US-A- 5 893 835
- US-A- 5 954 736
- US-A- 6 129 735
- US-B1- 6 214 023

## Description

### FIELD OF THE INVENTION

The present invention relates, in general, to an improved tissue pad and blade for use in an ultrasonic surgical instrument, such as an ultrasonic clamp coagulator.

### BACKGROUND OF THE INVENTION

Ultrasonic surgical instruments are finding increasingly widespread applications in surgical procedures by virtue of the unique performance characteristics of such instruments. Depending upon specific instrument configurations and operational parameters, ultrasonic surgical instruments can provide substantially simultaneous cutting of tissue and hemostasis by coagulation, desirably minimizing patient trauma. The cutting action is typically effected by an end-effector at the distal end of the instrument, with the end-effector transmitting ultrasonic energy to tissue brought into contact therewith. Ultrasonic instruments of this nature can be configured for open surgical use, laparoscopic or endoscopic surgical procedures.

Ultrasonic surgical instruments have been developed that include a clamp mechanism to press tissue against the end-effector of the instrument in order to couple ultrasonic energy to the tissue of the patient. Such an instrument is disclosed in United States Patent no. 5,322,055.

Various configurations have been known for the ultrasonic end-effector of the above type of clamp coagulator apparatus. The various configurations optimize the manner in which tissue is coupled to the end-effector or blade, with particular attention paid to achieving the desired degree of tissue cutting and concomitant coagulation.

With current instrumentation surgeons may improve the speed of cutting with these devices by increasing the clamping force of the instrument but this lowers the amount of coagulation that is done to the tissue and thus lowers hemostasis. This effect is more dramatic at higher blade amplitudes for a given blade geometry. Achieving first-cut hemostasis with currently available ultrasonic instruments usually requires the surgeon to apply energy in one of a number of ways. In one instance, the surgeon may utilize different aspects of the blade (blunt and sharp surfaces). They first apply energy to the structure with the instrument in "blunt" mode, to coagulate the structure, and then to transect it with the "sharp" mode of the instrument. This is time consuming, therefore more advanced surgeons have adopted a second methodology that makes an improved cut by varying the pressure applied to the structure during the course of the energy application. Experience with current instrumentation has shown that lower application of pressure will coagulate the tissue structure while a higher application of pressure will transect the tissue structure. Though this method is faster and does give a first-cut hemostasis, it may at times be difficult to perform correctly and difficult to reproduce.

It has also been observed that ultrasonic devices may make an uneven cut when grabbing large bites of tissue. This occurs because the tip velocity of ultrasonic devices drops off sinusoidally as a function of the distance from the node to the tip. When a constant force is applied to tissue (homogeneous and isotropic) with a blade that has an energy profile that is sinusoidal, the energy delivered to the tissue has the same sinusoidal profile. This varying energy profile directly affects both the coagulation and cutting tissue effects and causes both of these tissue effects to vary depending upon the location of the tissue within the jaw.

In conventional ultrasonic medical devices, as for example, disclosed in US Patent No. 5,322,055, the tissue is pressed against the side of an active blade by a clamp arm or clamping device. In this configuration the tissue presents a frictional drag load to the resonant system. The frictional drag to the system is overcome as the generator applies more energy to the blade and tissue proportional to the frictional drag on the system. The tissue frictional drag is a function of at least two parameters, blade velocity and the applied force at the tissue/blade interface. In most systems the blade velocity is user selected at the generator and remains a constant throughout a single cut. The blade velocity, however, does vary along the length of the blade. In typical systems the blade velocity is greatest at the distal end of the blade and drops off roughly sinusoidal moving proximally to the first waveguide node. The force at the tissue/blade interface is created by the compression of the tissue to the blade, by the clamp arm, which is a function of the pressure applied by the surgeon at the instrument interface. Therefore, if an instrument could vary the compression exerted upon the tissue across the cross section in a single cut, it could control the amount of inflowing energy and therefore, the tissue bio-effect.

Compression is important because tissue is visco-elastic. Therefore when it is compressed between two structures, such as the ultrasound blade and the clamp arm, it will demonstrate both viscous and elastic properties.

Due to the viscous nature of the tissue it will flow out of the instrument jaws slightly. The elastic nature allows the tissue, when compressed, to act like a spring. This means that the force exerted by the tissue on both interfacing surfaces, clamp arm and instrument blade, is proportional to the distance that the tissue has been compressed. Therefore, as the compression distance of the tissue varies the energy delivered to the tissue varies and thus the achieved bio-effect varies. As the surgeon decreases the force of their grip the tissue is compressed a smaller distance and the energy delivered to the tissue is reduced, resulting in a reduced energy transfer during coagulation of the tissue. As the force and thus tissue compression are increased, the energy delivered to the tissue increases, and a cut is achieved. However, the cut will likely appear in the same vicinity as the coagulation, which may reduce the sealing effect. It would be desirable to provide a ultrasonic clamp coagulator to optimize the tissue effects discussed herein. The present invention is particularly directed to an improved clamp arm arrangement, including a tissue pad having a varying height surface. The tissue pad and blade of the present invention have been developed to address this desire.

International patent publication WO-A-99/35982 discloses a surgical tool for cutting and/or coagulating tissue which includes piezo-electric driver to generate ultrasonic energy comprising torsional mode vibrations. A waveguide is operatively connected at a proximal end to the driver and extends a distance of n lambda T/2 (where lambda T is the wavelength of ultrasonic vibration in the material of the work horn or waveguide). A distal end of the waveguide is provided with a cutting and/or coagulating tool.

### SUMMARY OF THE INVENTION

The invention relates to an end effector for an ultrasonic surgical instrument as defined in claim 1. Disclosed is an ultrasonic surgical instrument that combines end effector geometry to best affect the multiple functions of an ultrasonic clamp coagulator. These end-effectors contain a combination of specially shaped ultrasonic blades and tissue clamping pads that can be used in combination or separately and that control the amount of cutting and coagulation that occurs during use. These combinations accomplish this by controlling the amount of compression that the tissue sees as it is pressed against the active blade, leading to a custom coagulation and cut zone.

In particular the disclosure presents a compression zone designed to control the amount of energy delivered to a specific part of the tissue by varying the compression on the tissue with a single application of clamping force. Since the compression force is directly proportional to the distance of compression the disclosure features a clamp arm with a tissue interface pad having a varied height to control the tissue effect. By placing the cut zone directly between two coagulation zones, a zone of coagulation is created on each side of the cut, increasing the reliability of the seal. In an alternate embodiment the blade may comprise a tissue interface surface having a varied height to control the tissue effect.

In one embodiment the disclosure controls both the cutting zone and the coagulation zone in the form of a tissue pad having compression cross- section similar to a step. The highest portion of the pad causes more energy to be directed to the tissue and causes cutting, while the lower portion of the pad causes less compression and causes the tissue coagulation.

Alternatively, the tissue pad may have a varying cross-sectional height dimension instead of a step.

In an alternate embodiment, the dimensions of the tissue pad change from the distal end of the blade to the proximal end of the blade. In one embodiment the raised section of the tissue pad has a varying height from the distal end of the blade to the proximal end of the blade. Alternatively, the coagulation zone section of the pad has a varying height from the distal end to the proximal end of the blade. In another embodiment the width of the raised section of the tissue pad varies from the distal end to the proximal end of the tissue pad (or blade).

In still a further embodiment, a tissue pad with a continuously rounded tissue-contacting surface is opposed to a blade with a similar continuously rounded tissue-contracting surface such that when brought into contact, the

center sections of the tissue pad and blade contact to create a cut zone, while the remainder of the two parts create two coagulation zones on either side of the cut zone. These coagulation zones, by the curved nature of the tissue pad and blade generate zones with compression that decrease as a function of the distance from the cut zone. This enables an improvement over the stepped tissue pad design in that this embodiment is accommodating to a wider range of tissue thickness.

An embodiment of the disclosure, not claimed, employs a trough, or U-shaped clamping surface. This embodiment provides a much wider coagulation zone than conventional clamp/coagulator pad designs. The U-shaped clamping surface also insures that the tissue sample is "wrapped" to the ultrasonic blade in order to put the tissue in contact with the blade in compression mode, regardless of the instrument's orientation. Having the tissue cut surface in compression keeps the tissue in the jaw and allows for an improved sealing of tubular structures such as blood vessels.

As would be apparent to those skilled in the art, the present invention has, without limitation, application in conventional endoscopic and open surgical instrumentation as well as application in robotic-assisted surgery.

These and other features and advantages of the present invention will become apparent from the following more detailed description, when taken in conjunction with the accompanying drawings which illustrate, by way of example, the principles of the invention.

### BRIEF DESCRIPTION OF THE DRAWINGS

The invention and its preferred embodiments are defined in the appended claims. The invention itself, however, both as to organization and methods of operation, together with further objects and advantages thereof, may best be understood by reference to the following description, taken in conjunction with the accompanying drawings in which:

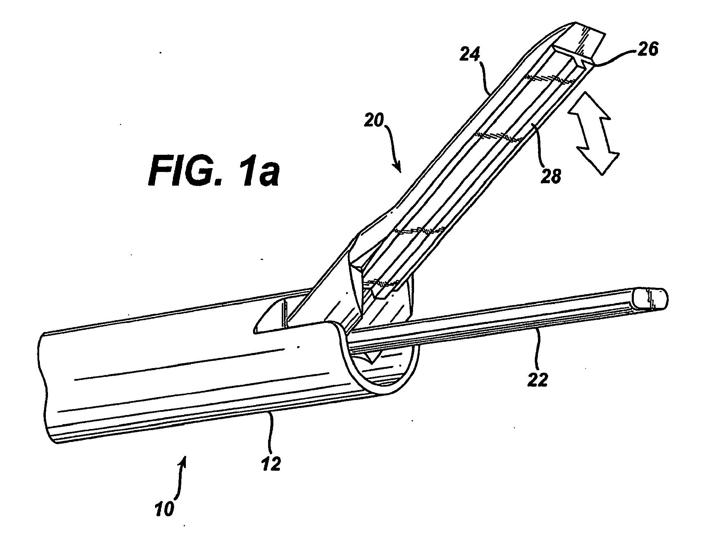

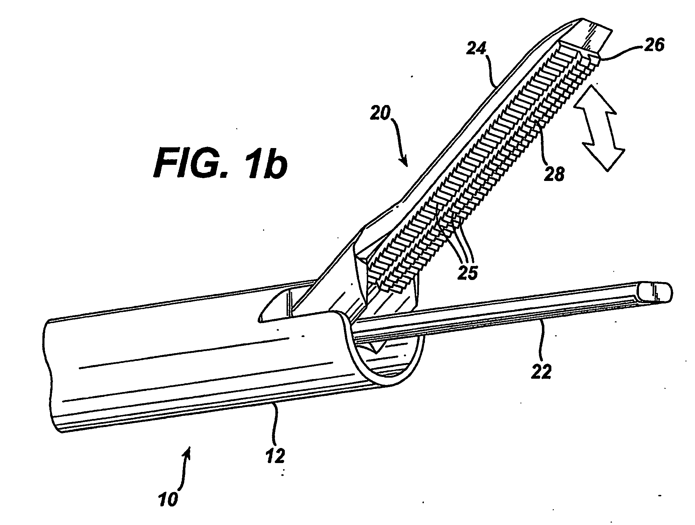

FIGURE 1 a is a perspective view of an ultrasonic end-effector having a clamp tissue pad with a raised surface;
FIGURE 1 b is a perspective view of an ultrasonic end-effector and an alternate embodiment of a clamp tissue pad with a raised surface;
FIGURES 2-5 are cross-sectional views of the blade and alternate embodiments of the tissue pad;
FIGURE 6 is a perspective view of an ultrasonic end-effector and an alternate embodiment of the tissue pad;
FIGURE 7 is a cross-sectional view of the tissue pad and blade of Figure 6;
FIGURES 8 and 9 are schematic representations of tissue compressed between a clamp pad and sharp-edged blade and resulting tissue effects;
FIGURES 10 and 11 are schematic representations of tissue compressed between a clamp pad and round-edged blade and resulting tissue effects;
FIGURES 12a-b are alternate embodiments of a clamp pad having a raised surface;
FIGURE 13 is a perspective view of an ultrasonic end-effector and an alternate embodiment of the tissue pad;
FIGURE 14 is a cross-sectional view of the tissue pad and blade of Figure 13;
FIGURE 15 is a schematic representation of the velocity change along the length of the blade;
FIGURE 16 is an elevation view of the tissue pad and blade of Figure 13;
FIGURE 17 is a cross-section view of an alternate embodiment, not claimed, of the blade in cooperation with a "U"-shaped clamp pad;
FIGURE 18 is a cross-section view of an alternate embodiment of a "U"-shaped clamp pad in cooperation with the blade of Figure 17;
FIGURES 19-20 are schematic representations of the tissue effects dependent upon the position of the blade; and
FIGURES 21-22 are schematic representations of the tissue effects in conjunction with the embodiment, not claimed, of Figure 17.

### DETAILED DESCRIPTION OF THE INVENTION

The present invention will be illustrated in the form of a straight blade. The invention has equal application in ultrasonic devices having curved blades.

Figure 1 shows an end-effector 20 of an ultrasonic clamp/coagulation medical instrument. Shown in the figure is the distal end of the instrument 10 including; the instrument shaft 12, the ultrasonic blade 22, which extends out of the instrument shaft 12, the movable clamp arm 24, which pivots with the instrument shaft in the direction shown. The clamp arm 24 includes a tissue pad 26, preferably formed from Teflon or other suitable low-friction materials, which is mounted for cooperation with the blade 22. With this construction, tissue is grasped between the tissue pad 26 and the blade 22.

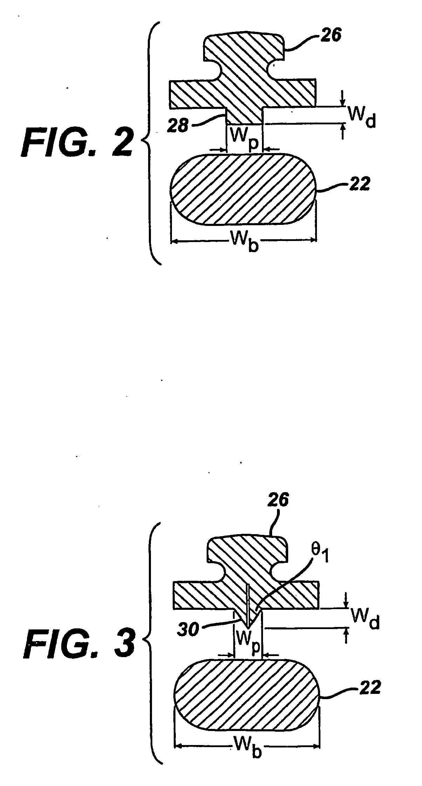

Figure 2 shows a cross section of the tissue pad 26 and the ultrasonic blade 22. This cross section illustrates the three important dimensions of the above device; Wb, Wp, and Wd. Wb is the overall width of the blade itself, and Wp is the width of the raised portion or energy director 28 of the tissue pad 26. Ideally the ratio of Wp to Wb would be some value less than one that would determine the ratio of cutting to coagulation that would occur when the instrument is in use. The preferred range of the ratio of Wp to Wb would be less than about 1: 2; however, the dimension of Wp may be as low as 0.001 inches. Wd is also very important because it determines the ratio of energy application between the tissue under the raised clamp portion 28 and the tissue under the remainder of the blade width. The higher the value of Wd, the less coagulation will occur in the zone of tissue on either side of the raised portion 28. The ratio of Wd to Wp is preferred in the range of greater than 1:4 and less than 2:1. However, more importantly is the ratio of Wd to the anticipated tissue thickness. Wd needs to be less than the overall thickness of the tissue being transected, thus applying pressure in the coagulation zone as well as the cut zone.

As is well known to those skilled in the art, the clamp pad 26 and raised portion 28 may be modified to include in combination or individually gripping teeth 25 to enhance the tissue-gripping capabilities of the end-effector as shown in Fig. 1b. Teeth 25 may be arranged as disclosed in U.S. patent no. 6,068,647.

Figures 3 through 5 show alternate embodiments of the tissue pad 26 and blade 22 for use with the ultrasonic clamp/coagulation instrument 10 of Fig. 1 with like reference numerals having the same description as given in Fig. 1. Fig. 3 illustrates the tissue pad 26 having a raised clamp portion, or energy director, 30 having a triangular cross section. The parameters Wb, Wp and Wd define the same dimensions as in Fig. 2, but the raised clamp area is further defined by the angle Θ₁. This angle defines a wedge shaped area that would increase cutting speed and would make a thinner cut. The only limitation on the value of angle Θ₁ is that the resulting energy director is not so thin as to be structurally unsound.

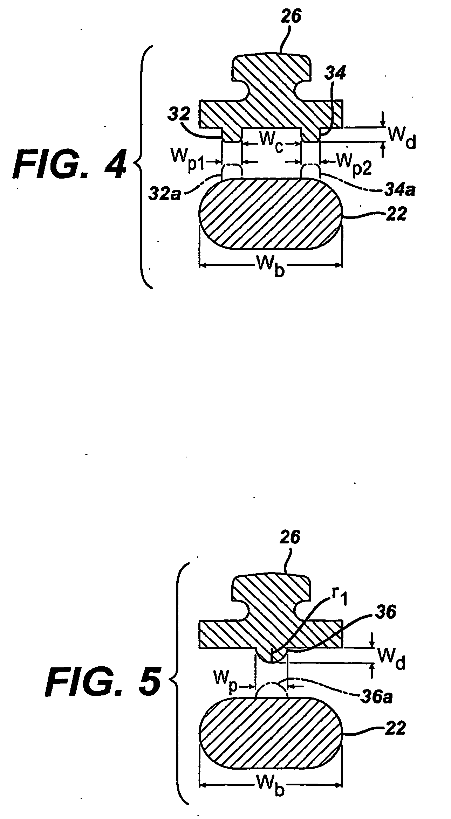

Figure 4 illustrates a tissue pad 26 having two energy directors and a separation distance Wc. Also shown are the critical parameters Wp1 and Wp2, (width of energy directors 32 and 34, respectively), Wd and Wb. In this embodiment, the energy directors allow the instrument to make multiple cuts of a tissue sample at the same time. This could allow a tissue structure, such as a fallopian tube, to be sealed and ligated and a sample of the tube to be removed. In the case of vessels this embodiment could be used to place a double seal on a vessel. As in previous embodiments, the ratio of Wp1+Wp2 to Wb would determine the ratio of cut tissue verses coagulated tissue and would be similar to the ratios previously discussed. The parameter Wc controls the amount of tissue between the two cuts defined by Wp1 and Wp2. Dimensions of Wp1 and Wp2 are similar to previous embodiments, but Wc would be about twice Wp in order to see any effect of spacing, that is, if a sample of tissue needs to be removed.

Figure 5 shows a partial cross section of the tissue pad 26 and the ultrasonic blade 22 and an energy director 36. Dimensions Wb, Wp and Wd define the same dimensions as in Figure 2, but the raised clamp area 36 is further defined by the radius r1. This radius defines the raised tissue pad section that would give a faster cut than in the embodiment in Figure 1 but slower than in Figure 3. It also would have a wider ratio of cut area to coagulation area. Although Fig. 5 shows the center of r1 to be aligned such that r1 is exactly twice Wp, it is also possible for the radius to be offset from this position such that the curve subscribes only a portion of a full diameter. This would allow for radii larger than twice Wp to be used.

Figs. 4 and 5 also illustrate alternate energy directors that are incorporated onto the blade 22. In Fig. 4, energy directors 32a and 34a are shown in phantom on blade 22 in direct opposition to energy directors 32 and 34. It is possible to use energy directors 32a and 34a alone and in cooperation with presently available tissue pads as disclosed in the cited prior art references; alternatively energy directors 32a and 34a may be used in combination with energy directors 32 and 34. Energy director 36a is shown in Fig. 5 and can be use alone or in combination with energy director 36. The energy directors located on the blade 22 may be manufactured during the machining process of blade 22.

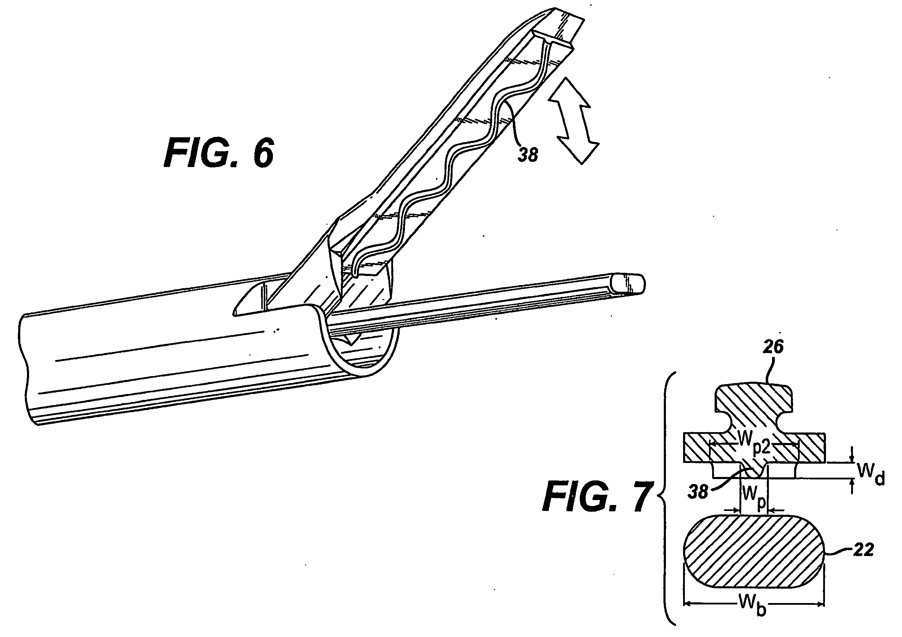

A further embodiment of the invention is shown in Figs. 6 and 7 with like reference numerals having the same description as Fig. 1. In this embodiment there is a single energy director 38, but it is deployed in a nonlinear fashion, (ie. curvy path) from the distal end of tissue pad 26 to the proximal end of tissue pad 26. Fig. 7 illustrates the critical parameters Wb, Wp, Wp2, and Wd. Wb is the width of the blade 22 and determines the overall affected area of the tissue. Wd is the height of the energy director 38 and determines the ratio of pressure difference between the cut zone and the coagulated zone. Wp is the width of the energy director and the ratio of Wp to Wb determines the ratio of coagulated tissue to cut tissue. The parameter Wp2 determines the spread of the path of the energy director across the Wb dimension. Preferably, Wp2 is about two times Wp and less than Wb. The embodiment illustrated in Fig. 6 has equal application for the previously disclosed embodiments of the invention.

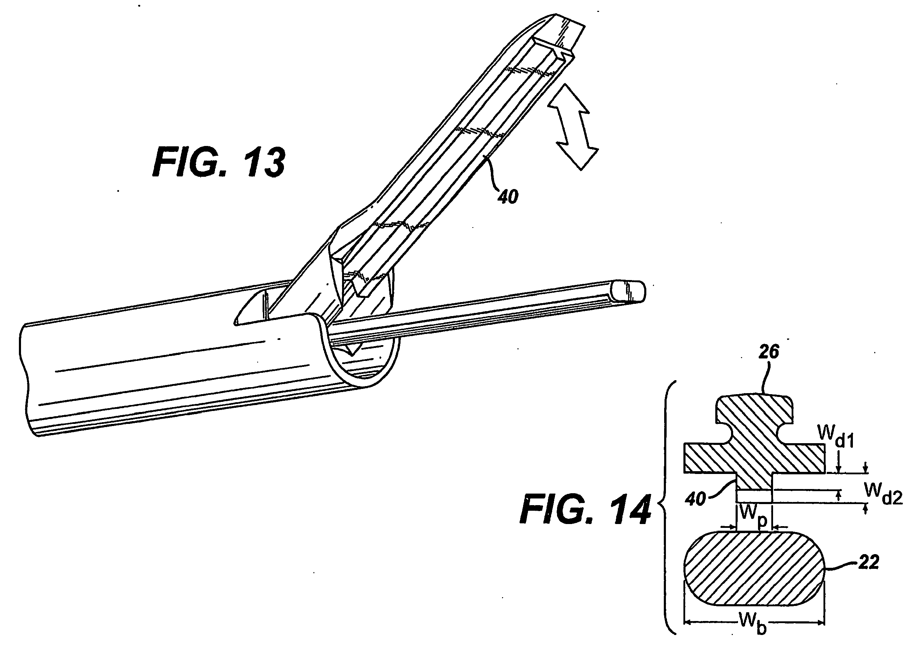

A further embodiment of the invention is shown in Figures 13 through 16 with like reference numerals having the same description as Fig. 1. In this embodiment, the raised portion, or energy director, 40 has a varying dimension from its distal to proximal end. Figure 14 illustrates the critical dimensions of the ultrasonic blade and tissue pad, Wb, Wp, Wd1 and Wd2. Wb is the width of the ultrasonic blade and determines the amount of tissue that is affected by the device. Wp is the width of the energy director and the ratio of Wp to Wb determines the ratio of the coagulated tissue to the cut tissue when the device is used. Wd1 shows the height of the energy director 40 at its distal end while Wd2 shows the height of the energy director 40 at the proximal end of the tissue pad 26. Wd2 is always larger than Wd1 and the height of the energy director 40 changes linear from Wd1 to Wd2. As is obvious to those skilled in the art, the height of the energy director 40 may also change in a nonlinear fashion.

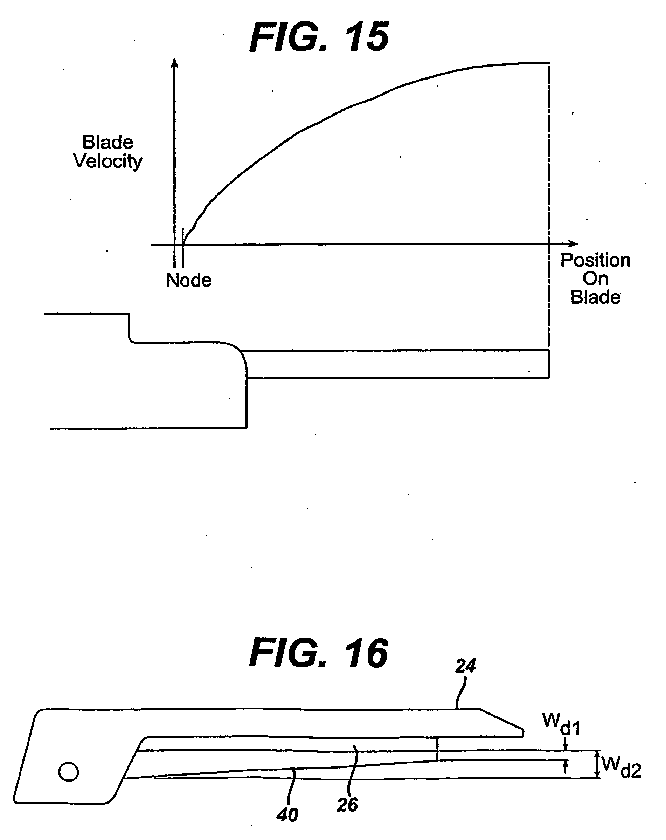

Figure 15 shows a side view of an exemplary end-effector of an ultrasonic clamp / coagulation device with the clamp arm and tissue pad removed for ease of illustration. The graph displays how the velocity of the end-effector varies along the length of the end-effector. Specifically, the end-effector velocity progresses in a sinusoidal fashion, (zero at the node and maximum at the most distal tip of the end-effector). Figure 16 shows a side view of the clamp arm 24, tissue pad 26 and energy director 40 shown in Figs. 13 and 14 and illustrates the dimensions Wd1 and Wd2 and shows the transition of the height of the energy director as it progresses from the distal end of the tissue pad to the proximal end of the tissue pad in a nonlinear fashion. This transition creates a curved energy director surface that is proportional to the drop off in tip velocity shown in the graph in Figure 15, so that as the tip velocity drops off, the height of the energy director increases, thus keeping constant energy delivered to the tissue.

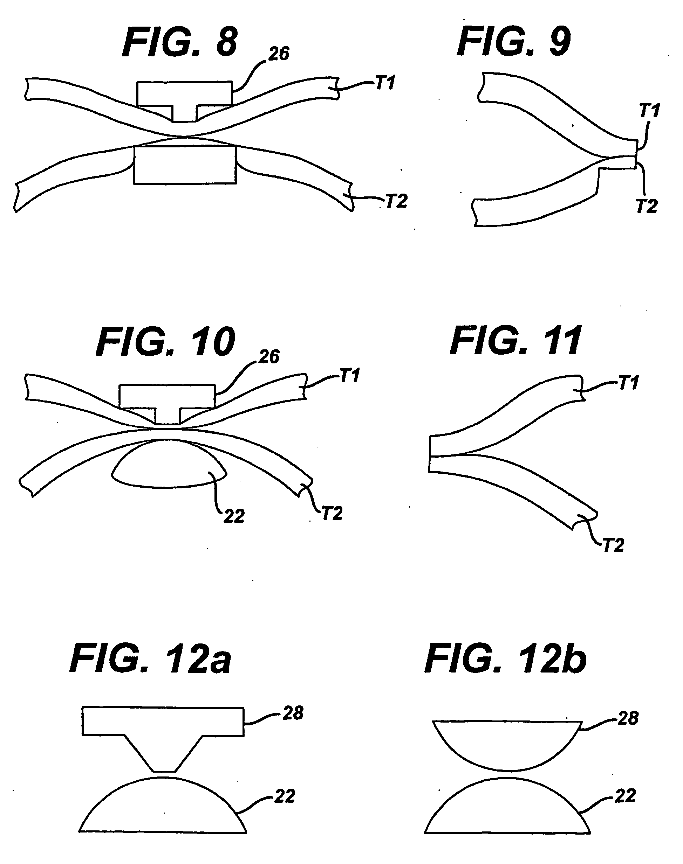

Preferably, the embodiments of Figs. 1 through 7 and 14 are used in conjunction with a blade 22 having a rounded cross section. Figure 8 shows the cross section of the distal end of an ultrasonic clamp/coagulation device as it is compressing a vessel or tubular structure in order to divide the tissue and seal both ends of the divided tissue. As the tissue pad 28 and an ultrasound blade 22, having discrete edges, are brought closer together by pivoting the clamp arm (not shown), the walls of the tissue, T1 and T2 are brought into contact with each other and compressed together. As energy is applied to the tissue through the ultrasound blade 22 and directed by the energy director 28 of Fig. 2 the two walls, T1 and T2, are coagulated and cut. Figure 9 shows a cross section of the left hand side of the tissue from Fig. 8 after it has been coagulated and divided. A defect in the tissue weld is created due to the visco-elastic properties of the tissue and the sharp corner of the ultrasound blade. This tissue defect causes wall T2 to be thinned, thus weakening the tissue weld and in the case of vessels, leading to lower burst pressure ratings on the seal. Figure 10 shows the preferred embodiment of the distal end of an ultrasonic clamp/coagulation device as it is compressing a vessel or tubular structure in order to divide the tissue and weld it. In this embodiment the ultrasound blade has a rounded cross section and does not create sharp corners as in Figure 8. In addition as the pressure is applied to the tissue during transection, the high pressure section in the cut zone pushes the coagulum created during the cut to the lower pressure areas in the coagulation zones, which in turn push the coagulum into the uncompressed lumen of the vessel. This coagulum can then cool and form a seal or plug in the lumen that increases the effectiveness of the seal.

Figure 11 shows a cross section of the right side of the tissue shown in Figure 10 after energy has been applied to it and it has been divided and coagulated. Because of the shape of the ultrasound blade there is no tissue defect 1 and therefore no weak spot.

Figures 12a and 12b illustrate alternate embodiments of an energy director 28 having a raised area in combination with a curved blade 22 that would provide the tissue effects shown in Fig. 11. Fig. 12a shows a trapemidal-shaped energy director 28 section, which provides for varying compression as a function of the distance from the cut zone. Both embodiments are more robust over a broader range of tissue thickness.

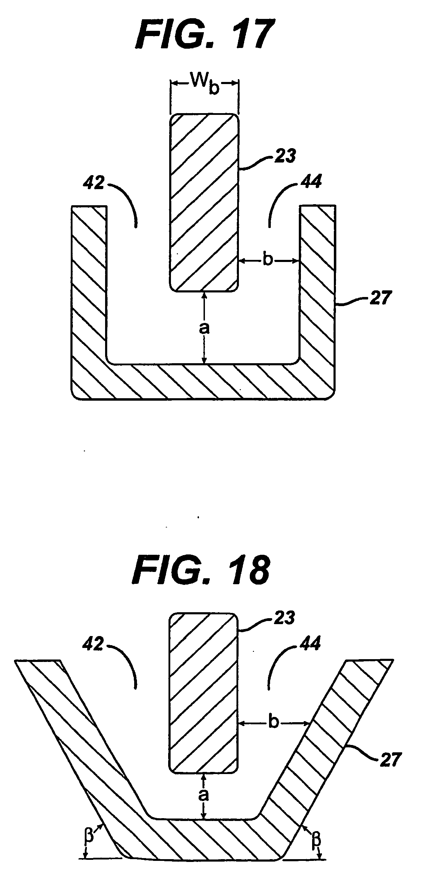

Figures 17 and 18 illustrate a tissue pad 27 and blade 23, not claimed, useful in conjunction with the ultrasonic cut/coagulation instrument 10. In this embodiment the tissue pad 27 is U-shaped and having the parameters a and b, and the ultrasonic blade is rectangular in shape and having the critical parameter Wb. The ratio of the parameters a to b determine the ratio of energy delivery to tissue that is directly under the blade as opposed to compressed in the side slots 42 and 44. The parameter Wb, determines the amount of tissue that is cut as opposed to coagulated. The sides of the tissue pad would help "wrap" the tissue around the ultrasonic blade in order to create larger coagulation zones as opposed to previous embodiments. In

Fig. 18, the U-shaped tissue pad 27 has a complex geometry that includes the angle β. This embodiment would allow the value of parameter b to vary, or increase, as you move vertically along the sidewalls of the tissue pad. This would lower the amount of energy dissipated into these regions, thus causing the amount of coagulation to decrease. The value of angle β would be a matter of design choice depending on the amount of coagulation needed.

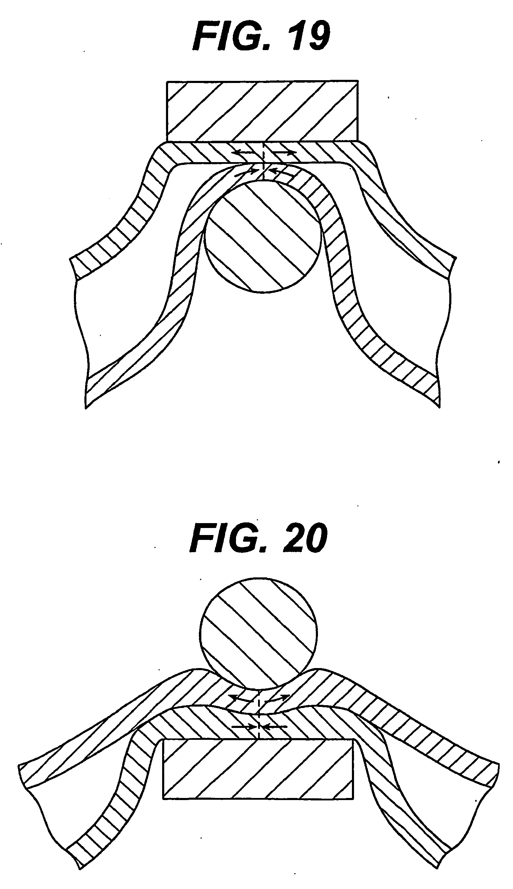

The benefit of the U-shaped tissue pad is best understood by examination of the tissue effects when the tissue is compressed between the tissue pad and ultrasonic blade. Referring to Figure 19, a tubular tissue sample is compressed in the between a blade 22 and tissue pad 26 in an "upward" fashion, that is, with the tissue pad 26 on the top. In this configuration, the clamping surface of the tissue is above the cutting surface of the tissue. Due to gravity, the tissue droops down to either side of the ultrasonic blade 22 and asserts a bending force to the tissue structure. This causes the top wall, or the clamping surface, to be in a tensile load and the bottom wall, or cutting surface to be under a compressive load. As the ultrasonic blade works it's way through the tissue the cutting surface would remain in the jaw due to the compressive forces, allowing the two walls to remain in intimate contact throughout the coagulation process and thus creating a better seal.

Figure 20, on the other hand, shows a cross section of the tubular tissue as it is compressed in the jaws with the jaws in a "downward" orientation, that is, the tissue pad on the bottom. In this figure the cutting surface of the tissue is above the clamping surface of the tissue. In this configuration the tissue would have the cutting surface on the top of the bending load, thus applying a tensile force to the tissue as it is cut Since tissue is visco-elastic, it would snap out of the jaw as it is cut, thus shortening the time that the walls are compressed in the coagulation zone and weakening the seal of the structure.

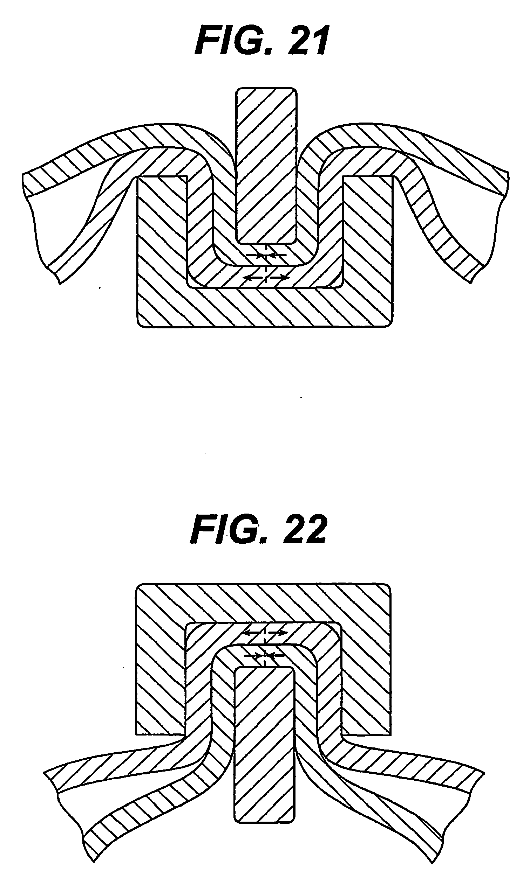

Figures 21 and 22 both show a cross section of the U-shaped tissue pad and tissue compressed therein. Figure 21 shows the instrument in the "downward" position with the tissue pad on the bottom and Figure 22 shows the instrument in the "upward" position with the tissue pad on the top. Figures 21 and 22 both show that the cutting surface of the tissue is in the compression side regardless of the orientation of the instrument. The U-shaped tissue pad forces an oriented bending load onto the tissue that is not affected by gravity. Therefore the tissue in contact with the ultrasonic blade is always in the compressive zone, even if the instrument is turned sideways.

## Claims

1. An end effector (20) for an ultrasonic surgical instrument (10), said end effector comprising:
an ultrasonic blade (22) having a proximal and a distal end;
a clamp arm (24) having a proximal and a distal end and configured for movement with respect to the blade;
the clamp arm having a clamping surface (26) and positioned for clamping tissue between the clamping surface and the blade;
the clamping surface defining at least one longitudinal axis, the longitudinal axis defining a raised clamping surface (28) which causes cutting, and the raised clamping surface (28) has a first dimension (Wd2) at the proximal end of the clamp arm (24) and a second dimension (Wd1) at the distal end of the clamp arm (24); and said first dimension (Wd2) is larger than the second dimension (Wd1).

2. The end effector of claim 1, wherein the cross section of the raised clamping surface is a rectangle.

3. The end effector of claim 1, wherein the cross section of the raised clamping surface is a triangle.

4. The end effector of claim 1, wherein the cross section of the raised clamping surface is rounded.

5. The end effector of claim 1, wherein the cross section of the clamping surface is rounded.

6. The end effector of claim 1, wherein the raised clamping surface is nonlinear with respect to the at least one longitudinal axis.

7. The end effector of claim 6, wherein the cross section of the raised clamping surface is a rectangle.

8. The end effector of claim 1, wherein the blade has a rounded cross section.

9. The end effector of claim 1, wherein the clamping surface defines two longitudinal axes, and each longitudinal axis defines a raised clamping surface.

## Patentansprüche

1. Endeffektor (20) für ein chirurgisches Ultraschallinstrument (10), wobei der Endeffektor Folgendes umfasst:
eine Ultraschallklinge (22) mit einem proximalen und einem distalen Ende;
ein Klammerarm (24) mit einem proximalen und einem distalen Ende, der relativ zur Klinge bewegt werden kann;
wobei der Klammerarm eine Klemmfläche (26) aufweist und zum Klammern von Gewebe zwischen der Klemmfläche und der Klinge angeordnet ist;
wobei die Klemmfläche zumindest eine Längsachse definiert, wobei die Längsachse eine erhabene Klemmfläche (28) definiert, die zum Schneiden führt und wobei die erhabene Klemmfläche (28) am proximalen Ende des Klammerarms (24) eine erste Abmessung (Wd2) und am distalen Ende des Klammerarms (24) eine zweite Abmessung (Wd1) aufweist; und wobei diese erste Abmessung (Wd2) größer ist als die zweite Abmessung (Wd1).

2. Endeffektor nach Anspruch 1, worin der Querschnitt der erhabenen Klemmfläche rechteckig ist.

3. Endeffektor nach Anspruch 1, worin der Querschnitt der erhabenen Klemmfläche dreieckig ist.

4. Endeffektor nach Anspruch 1, worin der Querschnitt der erhabenen Klemmfläche abgerundet ist.

5. Endeffektor nach Anspruch 1, worin der Querschnitt der Klemmfläche abgerundet ist.

6. Endeffektor nach Anspruch 1, worin die erhabene Klemmfläche relativ zu der zumindest einen Längsachse nicht-linear ist.

7. Endeffektor nach Anspruch 6, worin der Querschnitt der erhabenen Klemmfläche rechteckig ist.

8. Endeffektor nach Anspruch 1, worin die Klinge einen abgerundeten Querschnitt aufweist.

9. Endeffektor nach Anspruch 1, worin die Klemmfläche zwei Längsachsen definiert und jede Längsachse eine erhabene Klemmfläche definiert.

## Revendications

1. Effecteur (20) d'extrémité pour un instrument (10) chirurgical ultrasonore, ledit effecteur d'extrémité comportant :
une lame (22) ultrasonore présentant des extrémités proximale et distale ;
une branche (24) de clamp présentant des extrémités proximale et distale et configurée pour se déplacer par rapport à la lame ;
la branche de clamp présentant une surface (26) de clampage et étant positionnée pour clamper des tissus entre la surface de clampage et la lame ;
la surface de clampage délimitant au moins un axe longitudinal, l'axe longitudinal délimitant une surface (28) de clampage surélevée qui entraîne la découpe, et la surface (28) de clampage surélevée ayant une première dimension (Wd2) au niveau de l'extrémité proximale de la branche (24) de clamp et une seconde dimension (Wd1) au niveau de l'extrémité distale de la branche (24) de clamp ; et ladite première dimension (Wd2) étant supérieure à la seconde dimension (Wd1).

2. Effecteur d'extrémité selon la revendication 1, dans lequel la section transversale de la surface de clampage surélevée est un rectangle.

3. Effecteur d'extrémité selon la revendication 1, dans lequel la section transversale de la surface de clampage surélevée est un triangle.

4. Effecteur d'extrémité selon la revendication 1, dans lequel la section transversale de la surface de clampage surélevée est arrondie.

5. Effecteur d'extrémité selon la revendication 1, dans lequel la section transversale de la surface de clampage est arrondie.

6. Effecteur d'extrémité selon la revendication 1, dans lequel la surface de clampage surélevée n'est pas linéaire par rapport audit au moins un axe longitudinal.

7. Effecteur d'extrémité selon la revendication 6, dans lequel la section transversale de la surface de clampage surélevée est un rectangle.

8. Effecteur d'extrémité selon la revendication 1, dans lequel la lame a une section transversale arrondie.

9. Effecteur d'extrémité selon la revendication 1, dans lequel la surface de clampage délimite deux axes longitudinaux, et chaque axe longitudinal délimite une surface de clampage surélevée.
